# EUROPEAN PATENT SPECIFICATION

(11) **EP 1 759 675 B1**
(45) Date of publication and mention of the grant of the patent: **30.07.2008**
(21) Application number: 06119079.9
(22) Date of filing: 17.08.2006
(51) Int. Cl.: A61F 9/007, A61B 17/22, A61B 17/32

(54) **Nozzle for a surgical irrigating handpiece**
Düse für chirurgisches Spülhandstück
Buse pour pièce à main de rinçage chirurgicale

(30) Priority: 29.08.2005 US 214409
(43) Date of publication of application: 07.03.2007
(73) Proprietor: Alcon, Inc., 6331 Hünenberg (CH)
(72) Inventor: Kao, Daniel J., Lake Forest, CA 92630 (US); Ghannoum, Ziad R., Trabuco Canyon, CA 92679 (US); Sussman, Glenn R., Laguna Niguel, CA 92677 (US)
(74) Representative: Hanna, Peter William Derek

(56) References cited:
- EP-A1- 1 388 331
- DE-U1-4202004 014 10
- US-A- 4 024 866
- US-A- 5 254 106
- US-A- 5 788 667
- US-A1- 2002 177 802
- US-A1- 2005 277 898

## Description

### Background of the Invention

This invention relates generally to the field of cataract surgery and more particularly to an irrigating handpiece tip for capsular clean up following lens removal.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, the majority of cataractous lenses are removed by a surgical technique called phacoemulsification. During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

U.S. Patent No. 6,579,270 B2 (Sussman, et al.) discloses a surgical handpiece and tip having two coaxial tubes or channels mounted within a body. The first tube is used for aspiration and is smaller in diameter than the second tube so as to create an annular passage between the first and second tube. The annular passage communicates with a pumping chamber formed between two electrodes. The pumping chamber works by boiling a small volume of the surgical fluid. As the fluid boils, it expands rapidly, thereby propelling the liquid downstream of the pumping chamber out of the annular passage. The distal end of the annular gap is sealed by a nozzle at the distal ends of the first and second tube and a plurality of orifices or ports may be formed in the nozzle. As the expanding gas is propelled down the annular gap, the gas/liquid stream is forced out of the distal orifice in a controlled and directed manner. However, aspiration and irrigation flow patterns different that those described in this patent are sometimes desired, such as during cortical clean up or posterior capsule washing or lavage.

Therefore, a need continues to exist for a simple surgical handpiece and tip suitable for capsular clean up following lens removal.

### Brief Summary of the Invention

The present invention improves upon the prior art by providing a tip nozzle for a surgical irrigating handpiece. The nozzle produces a fan-like dispersion pattern when pressurized fluid is ejected out of the nozzle. The nozzle is particularly useful during the irrigation/aspiration ("I/A") portion of a cataract surgical procedure.

Accordingly, one objective of the present invention is to provide a tip nozzle for a surgical handpiece.

Another objective of the present invention is to provide a tip nozzle for a handpiece having a fan-like dispersion pattern.

Another objective of the present invention is to provide a tip nozzle for a surgical handpiece suitable for use during the I/A portion of a lens removal surgical procedure.

These and other advantages and objectives of the present invention will become apparent from the detailed description and claims that follow.

### Brief Description of the Drawings

FIG. 1 is a perspective view of the handpiece tip nozzle of the present invention.
FIG. 2 is a top plan view of the handpiece tip nozzle of the present invention.
FIG. 3 is a cross-sectional view of the tip nozzle taken at line 3-3 in FIG. 2.

### Detailed Description of the Invention

Handpieces suitable for use with the present invention include the INFINITI® AQUALASE® surgical system which is commercially available from Alcon Laboratories, Inc., Fort Worth, Texas. This system uses a tip generally described in FIG. 23 and 24 and column 7, lines 33-45 ofU.S Patent No. 6,579,270 B2 (Sussman, et al.).As described in this patent, tip 900 may alternatively consist of outer tube 965 surrounding and coaxial with inner tube 967. Distal tip 902 of outer tube 965 is flared or belled so as to allow nozzle 905 to be inserted between outer tube 965 and inner tube 967. As best seen in FIG. 23, nozzle 905 contains fluid channel 907 that communicates with orifice 904. Nozzle 905 seals annular gap 969 between outer tube 965 and inner tube 967. Pressurized fluid flowing down annular gap 969 is forced into fluid channel 907 and out orifice 904.

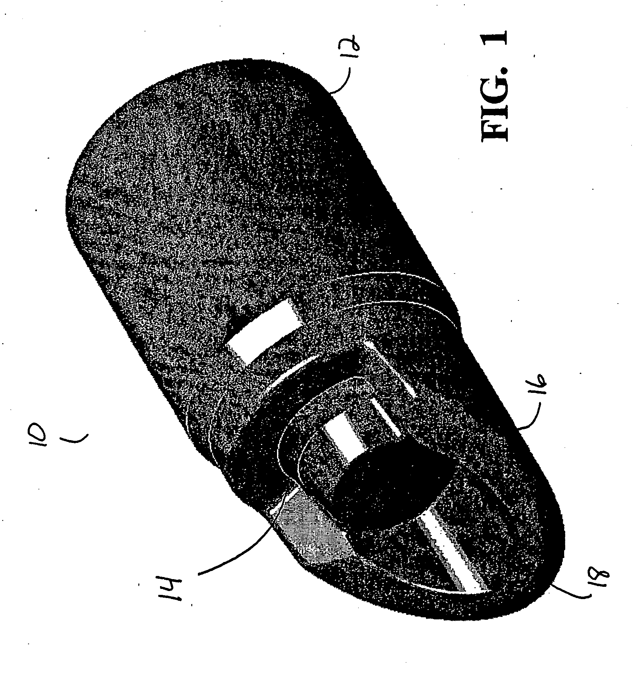

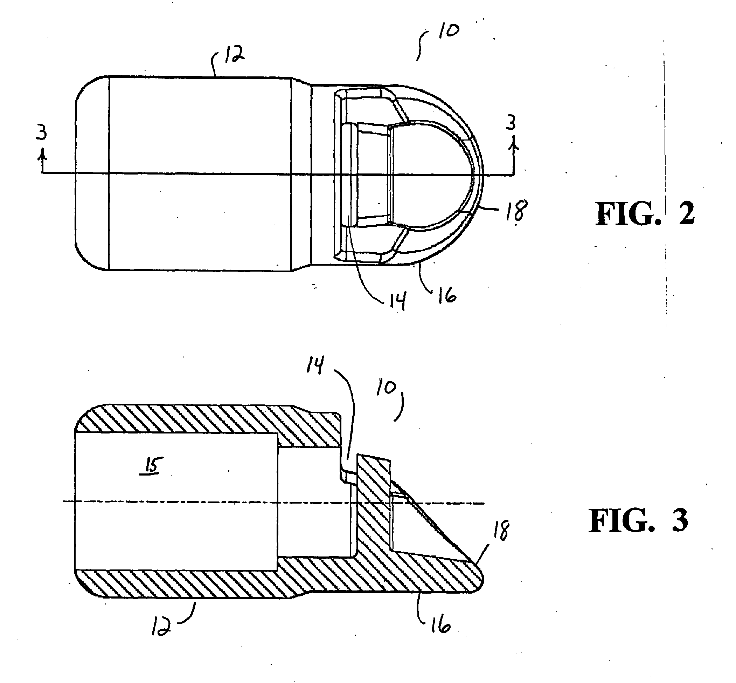

As best seen in FIGS. 1 and 2, tip nozzle 10 of the present invention generally includes body 12 having central bore 15 in fluid communication with arcuate irrigation port 14. Distal portion 16 of body 12 contains a rounded, elongated or scoop-shaped nose 18. The shape of nose 18 assists in inserting tip 10 into a surgical wound. Nozzle 10 may be of any suitable construction such as molded plastic or rubber with bore 15 sized and shaped to fit over the open shaft of an I/A handpiece (not shown) or may be formed directly on such shaft by conventional metal-working techniques.

Port 14 is sized and shaped so as to produce an evenly distributed, fan-like spray pattern when pressurized fluid is forced into bore 15 and out port 14. Preferably, port 14 is between 0.0508 mm (0.002 inches) and 0.0762 mm (0.003 inches) wide and cut over an arc of between approximately 120 and 140 degrees, with about 135 degrees being most preferred.

This description is given for purposes of illustration and explanation. It will be apparent to those skilled in the relevant art that changes and modifications may be made to the invention described above without departing from its scope as defined in the appended set of claims. For example, it will be recognized by those skilled in the art that the present invention may be combined with ultrasonic and/or rotating cutting tips to enhance performance.

## Claims

1. A tip nozzle(10) for a surgical irrigating handpiece, comprising:
a) a body (12), the body having a central bore (15); and
b) an irrigation port (14) in fluid communication with the bore,
wherein the body (12) further comprises a scoop-shaped nose (18)
**characterized in that**;
the irrigation port (14) is approximately between 0.0508 mm (0.002 inches) and 0.0762 mm (0.003 inches) wide and cut over an arc of between approximately 120 degrees and 140 degrees.

2. The tip nozzle of claim 1, wherein the irrigation port (14) is cut over an arc of about 135 degrees.

3. The tip nozzle of claim 1 or claim 2, which in use is adapted to produce a fan-like dispersion pattern when pressurized fluid is ejected out of the nozzle.

## Patentansprüche

1. Düse (10) an einer Spitze für ein chirurgisches Irrigationshandstück, die Folgendes aufweist:
a) einen Körper (12), wobei der Körper eine zentrale Bohrung (15) aufweist, und
b) eine Irrigationsöffnung (14), die in Fluidverbindung mit der Bohrung steht,
wobei der Köper (12) weiterhin eine schnabelförmige Nase (18) aufweist,
**dadurch gekennzeichnet, dass**
die Irrigationsöffnung (14) etwa zwischen 0,0508 mm (0,002 Zoll) und 0,0762 mm (0,003 Zoll) breit ist und über einen Bogen von etwa 120° bis 140° ausgeschnitten ist.

2. Düse an einer Spitze nach Anspruch 1, bei welcher die Irrigationsöffnung (14) über einen Bogen von etwa 135° ausgeschnitten ist.

3. Düse an einer Spitze nach Anspruch 1 oder 2, die dafür eingerichtet ist, im Gebrauch ein fächerartiges Verteilungsmuster zu erzeugen, wenn unter Druck gesetztes Fluid aus der Düse ausgestoßen wird.

## Revendications

1. Buse d'extrémité (10) pour pièce à main chirurgicale d'irrigation, comportant :
a) un corps (12), le corps ayant un alésage central (15), et
b) un orifice d'irrigation (14) en communication de fluide avec l'alésage,
le corps (12) comportant en outre un nez en forme de cuillère (18),
**caractérisée en ce que**,
l'orifice d'irrigation (14) est approximativement d'une largeur comprise entre 0,0508 mm (0,002 pouce) et 0,0762 mm (0,003 pouce), et découpé sur un arc compris entre environ 120 degrés et 140 degrés.

2. Buse d'extrémité selon la revendication 1, dans laquelle l'orifice d'irrigation (14) est découpé sur un arc d'environ 135 degrés.

3. Buse d'extrémité selon la revendication 1 ou 2 qui, en utilisation, est adaptée pour produire un motif de dispersion en forme d'éventail lorsqu'un fluide sous pression est éjecté hors de la buse.
